# EUROPEAN PATENT SPECIFICATION

(11) **EP 2 376 022 B1**
(45) Date of publication and mention of the grant of the patent: **23.01.2013**
(21) Application number: 09796191.6
(22) Date of filing: 15.12.2009
(51) Int. Cl.: A61F 2/01, A61F 2/97

(54) **OFFSET COUPLING REGION**
VERSETZTE VERBINDUNGSREGION
ZONE DE RACCORDEMENT DÉCALÉE

(30) Priority: 15.12.2008 US 122614 P; 27.08.2009 US 548499
(43) Date of publication of application: 19.10.2011
(73) Proprietor: Boston Scientific Scimed, Inc., Maple Grove, MN 55311 (US)
(72) Inventor: UTKE, Allen, Andover Minnesota 55304 (US); HENDERICKSON, Kyle, Litchfield Minnesota 55355 (US); EDELMAN, Peter, Maple Grove Minnesota 55311 (US); ARCAND, Ben, Minneapolis Minnesota 55406 (US); ANDERSON, James, Fridley Minnesota 55432 (US); RYAN, Ari, Sunnyvale California 94088 (US)
(74) Representative: Vossius & Partner
(86) International application number: PCT/US2009/068031
(87) International publication number: WO 2010/075075

(56) References cited:
- WO-A1-03/073961
- WO-A1-2007/033963
- US-A1- 2003 004 540
- US-A1- 2004 143 272
- US-A1- 2004 167 565
- US-A1- 2005 154 443

## Description

### Technical Field

This disclosure relates generally to an offset coupling region for use as part of a deployment system for the medical device.

### Background

Human blood vessels often become occluded or blocked by plaque, thrombi, other deposits, or material that reduce the blood carrying capacity of the vessel. Should the blockage occur at a critical place in the circulatory system, serious and permanent injury, and even death, can occur. To prevent this, some form of medical intervention is usually performed when significant occlusion is detected.

Several procedures are now used to open these stenosed or occluded blood vessels in a patient caused by the deposit of plaque or other material on the walls of the blood vessels. Angioplasty, for example, is a widely known procedure wherein an inflatable balloon is introduced into the occluded region. The balloon is inflated, dilating the occlusion, and thereby increasing the intraluminal diameter.

Another procedure is atherectomy. During atherectomy, a catheter is inserted into a narrowed artery to remove the matter occluding or narrowing the artery, i.e., fatty material. The catheter includes a rotating blade or cutter disposed in the tip thereof. Also located at the tip are an aperture and a balloon disposed on the opposite side of the catheter tip from the aperture. As the tip is placed in close proximity to the fatty material, the balloon is inflated to force the aperture into contact with the fatty material. When the blade is rotated, portions of the fatty material are shaved off and retained within the interior lumen of the catheter. This process is repeated until a sufficient amount of fatty material is removed and substantially normal blood flow is resumed.

In another procedure, stenosis within arteries and other blood vessels is treated by permanently or temporarily introducing a stent into the stenosed region to open the lumen of the vessel. The stent typically includes a substantially cylindrical tube or mesh sleeve made from such materials as stainless steel or nitinol. The design of the material permits the diameter of the stent to be radially expanded, while still providing sufficient rigidity such that the stent maintains its shape once it has been enlarged to a desired size.

Unfortunately, such percutaneous interventional procedures, i.e., angioplasty, atherectomy, and stenting, often dislodge material from the vessel walls. Some existing devices and technology use a filter for capturing the dislodged material from the bloodstream.

Document US 2005/154 443 discloses the preamble of claim 1.

### Summary of the Invention

Many procedures are performed percutaneously and transluminally using medical devices which are conveyed to the site of the intervention in a contained and/or constrained state which reduces their overall transverse dimension during insertion and transport. Once at the site, the constraint or containment is removed allowing the device to deploy. Related devices of the art employ an actuating member which is sharply curved or even doubly-curved which may lead to binding during the deployment process.

This disclosure relates to a medical device deployment system as disclosed in the appended claims comprising an elongated guide member having a proximal end and a distal end; a support segment attached near the distal end of the elongated guide member; a medical device disposed adjacent to the support segment; a containment element disposed about a portion of the medical device; an actuation element having a proximal end and a distal end and a first position and a second position, wherein the distal end of the actuation element engages a portion of the containment element in the first position and is at least partially disengaged from the containment element in the second position; and an offset coupling region between the guide member and the support segment, wherein the actuation element extends beyond the distal end of the guide member and generally coaxial with it to engage the containment element at one or more points in the first position.

Another aspect of the disclosure includes a medical device deployment system having an elongated guide member having a proximal end, a distal end, and a lumen associated therewith; an offset coupling region attached to the distal end of the guide member; an actuation element having a proximal end, a distal end, a first position, and a second position; a support segment having a distal end and having a proximal end attached to the offset coupling region, wherein the guide member, the support segment and the actuation element are generally coplanar; a medical device disposed adjacent to the support segment; a containment element disposed about at least a portion of the medical device, wherein the distal end of the actuation element engages at least a portion of the containment element in the first position and is at least partially disengaged from the containment element in the second position; and wherein the actuation element extends beyond the distal end of the guide member and extends generally coaxially with respect to the guide member to engage the containment element at one or more points in the first position.

A further aspect of the disclosure provides an example of a method of deploying a medical device comprising providing an elongated guide member having a proximal end and a distal end; providing a support segment attached near the distal end of the elongated guide member; providing a medical device disposed adjacent to the support segment; providing a containment element disposed about at least a portion of the medical device; providing an actuation element having a proximal end and a distal end and a first position and a second position, wherein the distal end of the actuation element engages at least a portion of the containment element in the first position and is at least partially disengaged from the containment element in the second position; providing an offset coupling region between the guide member and the support segment, wherein the actuation element extends beyond the distal end of the guide member and generally coaxial with it to engage the containment element at one or more points in the first position; advancing the medical device to a desired deployment site; and moving the actuation element from a first position to a second position thereby releasing the medical device from the containment element.

The following aspects are preferred embodiments of the invention for aspects 1 to 15 and examples for aspects 16 to 40.
1. A medical device deployment system comprising:
   an elongated guide member having a proximal end, a distal end, and a lumen at least partially therethrough;
   a support segment attached near the distal end of the elongated guide member;
   a medical device disposed adjacent to the support segment;
   a containment element disposed about at least a portion of the medical device;
   an actuation element having a proximal end and a distal end and a first position and a second position, wherein the distal end of the actuation element engages at least a portion of the containment element in the first position and is at least partially disengaged from the containment element in the second position; and
   an offset coupling region between the guide member and the support segment, wherein the actuation element extends beyond the distal end of the guide member and generally coaxial with it to engage the containment element at one or more points in the first position.
2. The medical device deployment system of aspect 1, wherein the support segment is generally parallel to and offset from the guide member.
3. The medical device deployment system of aspect 1, wherein the majority of the support segment is located distal of the distal end of the guide member.
4. The medical device deployment system of aspect 1, wherein the offset coupling region connects the guide member to the support segment.
5. The medical device deployment system of aspect 1, wherein the offset coupling region includes an aperture formed therein generally aligned with an axial extension of a lumen associated with the guide member,
   further wherein the actuation element passes through the lumen and the aperture.
6. The medical device deployment system of aspect 5, wherein the engagement between the actuation element in the first position and the containment element occurs generally along an axial extension of the lumen associated with the guide member.
7. The medical device deployment system of aspect 1, wherein the offset coupling region comprises a separate coupling element.
8. The medical device deployment system of aspect 7, wherein the separate coupling element is formed from at least one of metal, polymer, or reinforced polymer.
9. The medical device deployment system of aspect 7, wherein the offset coupling element comprises a first lumen adapted to receive the guide member and a second lumen adapted to receive the support segment.
10. The medical device deployment system of aspect 9, wherein one or both of the guide member and the support segment has a reduced cross-sectional area where it is received by the offset coupling element.
11. The medical device deployment system of aspect 1, wherein the offset coupling region comprises a double-curved portion of the guide member and the support segment comprises a further distal portion of the guide member.
12. The medical device deployment system of aspect 11, wherein support segment is generally parallel to the guide member.
13. The medical device deployment system of aspect 11, wherein the offset coupling region includes an aperture formed in the offset coupling region and generally aligned with an extended axis of a lumen associated with the guide member proximal of the offset coupling region,
   further wherein the actuation element passes through the lumen and the aperture formed in the offset coupling region.
14. The medical device deployment system of aspect 1, wherein the medical device is longitudinally constrained with regard to motion along the support section.
15. A medical device deployment system of aspect 1, wherein the actuation element is disposed at least partially within a lumen of the guide member and extends distally from the guide member in the offset coupling region.
16. The medical device deployment system of aspect 1, wherein the containment element has one or more apertures formed therein and the actuation element engages one or more apertures in the containment element in the first position and disengages from at least a majority of the one or more apertures in the containment element in the second position.
17. The medical device deployment system of aspect 1, wherein the containment element at least partially envelopes at least one of the medical device and the support segment when the actuation element is in the first position.
18. The medical device deployment system of aspect 1, wherein the containment element provides a constraining force to the medical device when the actuation element is in the first position.
19. The medical device deployment system of aspect 1, wherein the containment element does not provide a constraining force to the medical device when the actuation element is in the second position.
20. The medical device deployment system of aspect 1, wherein the offset coupling region between the guide member and the support segment is adapted for rotation about an axis generally coincident with the actuation member and passing through the distal end of the elongated guide member.
21. The medical device deployment system of aspect 1, wherein at least one of the guide member and the support segment has been partially relieved to enhance flexibility.
22. The medical device deployment system of aspect 21, wherein the partial relief comprises at least one of holes, slots and partial or complete grooves providing reduced diameter.
23. The medical device deployment system of aspect 22, wherein the holes, slots or grooves comprising the partial relief are uniformly spaced.
24. The medical device deployment system of aspect 22, wherein the holes, slots or grooves comprising the partial relief are nonuniformly spaced.
25. A medical device deployment system comprising:
   an elongated guide member having a proximal end, a distal end, and a lumen associated therewith;
   an offset coupling region attached to the distal end of the guide member;
   an actuation element having a proximal end, a distal end, a first position, and a second position;
   a support segment having a distal end and having a proximal end attached to the offset coupling region, wherein the guide member, the support segment and the actuation element are generally coplanar;
   a medical device disposed adjacent to the support segment;
   a containment element disposed about at least a portion of the medical device,
   wherein the distal end of the actuation element engages at least a portion of the containment element in the first position and is at least partially disengaged from the containment element in the second position; and
   wherein the actuation element extends beyond the distal end of the guide member and extends generally coaxial with respect to the guide member to engage the containment element at one or more points in the first position.
26. The medical device deployment system of aspect 25, wherein the medical device is at least one of a filter, a stent, or a grasping appliance.
27. The medical device deployment system of aspect 25, wherein the offset coupling region comprises at least one of metal, plastic, filled plastic, a tubular material, a solid material, an extension of the guide member, and an extension of the support segment.
28. The medical device deployment system of aspect 25, wherein the support segment is solid or hollow.
29. The medical device deployment system of aspect 25, wherein at least one of the guide member and the support segment has been partially relieved to enhance flexibility.
30. The medical device deployment system of aspect 29, wherein the partial relief comprises at least one of holes, slots and partial or complete grooves providing reduced diameter.
31. The medical device deployment system of aspect 30*,* wherein the holes, slots or 31. The medical device deployment system of aspect 30, wherein the holes, slots or grooves comprising the partial relief are uniformly spaced.
32. The medical device deployment system of aspect 30, wherein the holes, slots or grooves comprising the partial relief arc nonuniformly spaced.
33. The medical device deployment system of aspect 25, wherein the guide member comprises a lumen adjacent to the proximal end of the offset coupling region and a portion of the actuation member resides within the lumen.
34. The medical device deployment system of aspect 33, wherein the actuation member exits the guide member and the offset coupling region along a line generally coaxial with the lumen of the guide member.
35. The medical device deployment system of aspect 34, wherein the actuation member extends beyond the guide member and the offset coupling along a line generally parallel to the support segment.
36. The medical device deployment system of aspect 34, wherein the engagement between the actuation element in the first position and the containment element occurs generally along an axial extension of the lumen associated with the guide member.
37. The medical device deployment system of aspect 25, wherein the medical device is attached to the support segment and constrained to a limited range of motion along the support segment.
38. The medical device deployment system of aspect 25, wherein the medical device is at least partially disposed within the containment member and wherein the containment element has one or more apertures formed therein and the actuation element engages one or more apertures in the containment element in the first position and disengages from at least a majority of the one or more apertures in the containment element in the second position.
39. The medical device deployment system of aspect 1, wherein the containment element provides a constraining force to the medical device when the actuation element is in the first position and wherein the containment element does not provide a constraining force to the medical device when the actuation element is in the second position.
40. A method of deploying a medical device comprising:
   providing an elongated guide member having a proximal end and a distal end;
   providing a support segment attached near the distal end of the elongated guide member;
   providing a medical device disposed adjacent to the support segment;
   providing a containment element disposed about at least a portion of the medical device;
   providing an actuation element having a proximal end and a distal end and a first position and a second position, wherein the distal end of the actuation element engages at least a portion of the containment element in the first position and is at least partially disengaged from the containment element in the second position;
   providing an offset coupling region between the guide member and the support segment, wherein the actuation element extends beyond the distal end of the guide member and generally coaxial with it to engage the containment element at one or more points in the first position;
   advancing the medical device to a desired deployment site; and
   moving the actuation element from a first position to a second position thereby releasing the medical device from the containment element.

### Brief Description of the Figures

Figure 1 is a schematic side view of medical device deployment system of the prior art.
Figure 2A is a schematic side view of a medical device deployment system in a first position.
Figure 2B is a schematic side view of a medical device deployment system in a first position.
Figure 3 is a partial perspective view of a medical device deployment system in a first position.
Figure 4 is a schematic side view of a medical device deployment system in a second position.
Figure 5A is a schematic side view of a medical device deployment system in a first position.
Figure 5B is a schematic side view of a medical device deployment system in a first position.
Figure 6 is a schematic partial perspective view of a medical device deployment system in a first position.
Figures 7A-C represent alternate forms of an offset coupling region.

### Detailed Description

The following description should be read with reference to the drawings wherein like reference numerals indicate like elements throughout the several views. The drawings, which are not necessarily to scale, are not intended to limit the scope of the claimed invention. The detailed description and drawings illustrate example embodiments of the claimed invention.

All numbers are herein assumed to be modified by the term "about." The recitation of numerical ranges by endpoints includes all numbers subsumed within that range (e.g., I to 5 includes 1, 1.5, 2, 2.75, 3, 3.80, 4, and 5).

As used in this specification and the appended claims, the singular forms "a", "an", and "the" include the plural referents unless the content clearly dictates otherwise. As used in this specification and the appended claims, the term "or" is generally employed in its sense including "and/or" unless the content clearly dictates otherwise.

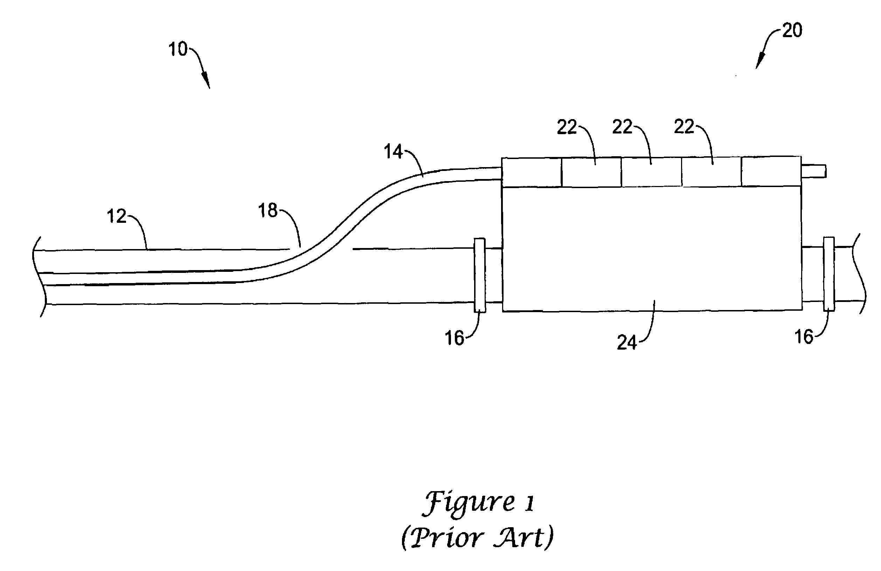

Figure 1 is a somewhat schematic side view of one type of filter deployment apparatus 20 of the prior art which is disposed in a distal region 10 of a catheter 12. Catheter 12 has a lateral opening 18 which allows a filter release wire 14 to exit the side of the catheter proximal of the distal end of the catheter. The wire 14 bends into an S-shaped form upon exiting the catheter 12 and then enters alternating loops 22 formed in a sheath 24 which is disposed about the filter (not shown within the sheath.) The release wire 14 may tend to bind as it exits the lateral opening 18 and at the loops 22. There may be some tendency for the wire 14 to deflect the distal end of the catheter 12, especially if the wire is stiff enough to resist lateral forces imposed by the loops 22. As may be seen, wire 14 is displaced laterally to a significant extent from a line defined by the extended axis of the catheter 12.

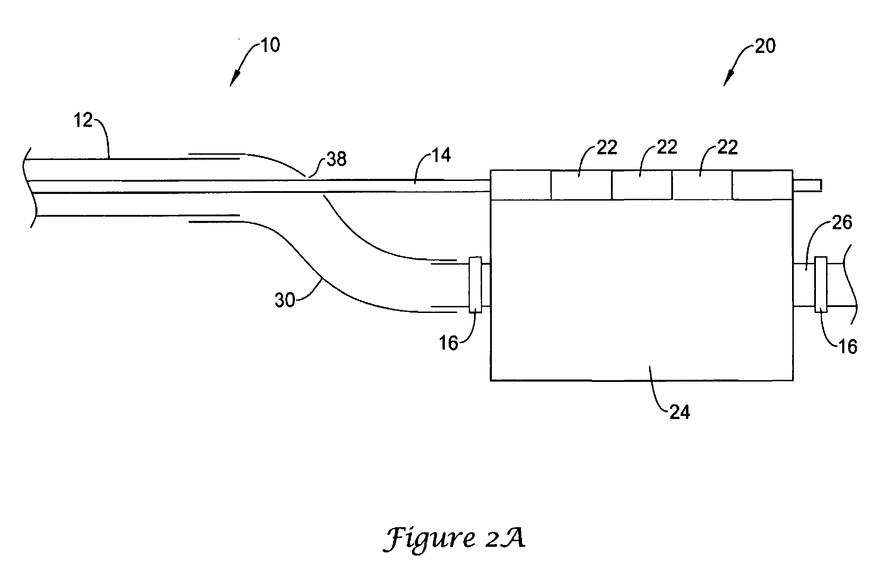

Figure 2A represents a medical device deployment system 20 in which an actuation element 14 passes through a lumen of guide member 12 and aperture 38 defined in offset coupling region 30 before entering apertures 22 formed in or by the containment element. The actuation element 14 passes from the guide member lumen and through the containment element in a generally straight line continuation of the guide member lumen. A medical device (not shown), which may be a filter, stent, a grasping appliance, or the like, is deployed about a support segment 26 attached to the guide member 12 by the intermediate offset coupling region 30 and any intervening couplings or other components. Although the following description primarily will relate to the deployment of a filter, it will be appreciated that other devices may be delivered and deployed in a similar manner. The support segment 26 may pass through, or adjacent to, the medical device. In some embodiments, the support segment 26 is somewhat longer than the medical device and may be configured to allow the deployed medical device to rotate or translate about or along a portion of the support segment. In such embodiments, the support segment 26 may include distal and proximal stops 16 which serve to confine the deployed medical device within a desired region of the support segment.

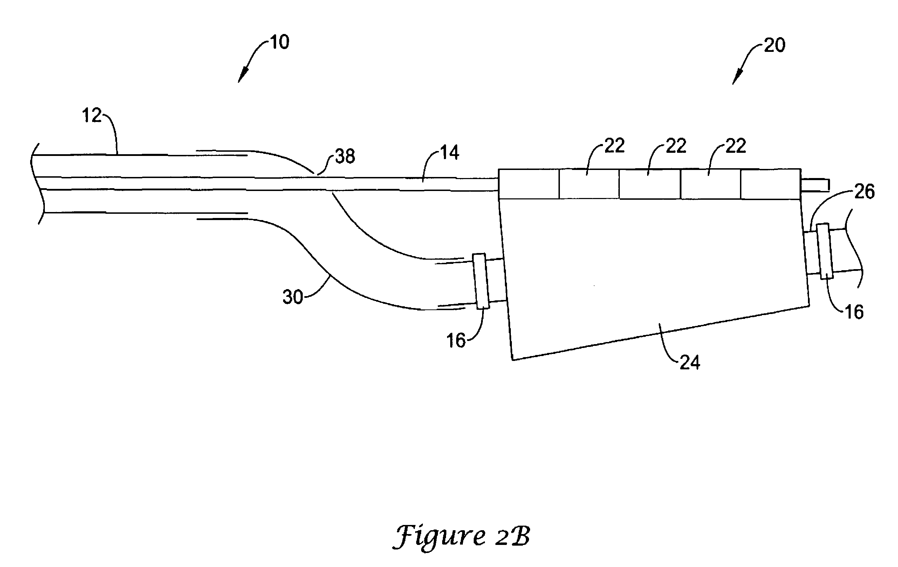

In other embodiments, the medical device may be completely released from the support segment or may remain rigidly affixed to the support segment 26 in the deployed state of the medical device. As illustrated in the embodiments of Figures 2A and 2B, the offset coupling region 30 is a separate element including proximal and distal lumens which receive the guide member 12 and the support segment 26 respectively. In other embodiments, one or both of the offset coupling region and the support segment may be formed together or from an extension of the guide member. The offset coupling region 30 may include a through lumen in fluid communication with the guide member 12 and the support segment 26 if desired. In some embodiments, the guide member 12, the offset coupling region 30, the support segment 26 and the actuation element may be substantially coplanar. In other embodiments, the guide member 12, the support segment 26 and the actuation element 14 may be substantially parallel. In yet other embodiments, the support segment 26 may form an acute angle with the actuation element 14 when lines corresponding to the axes of the guide member and the support segment are extended in either direction as necessary to reach a point of intersection. An embodiment illustrative of an angled support segment 26 may be found in Figure 2B.

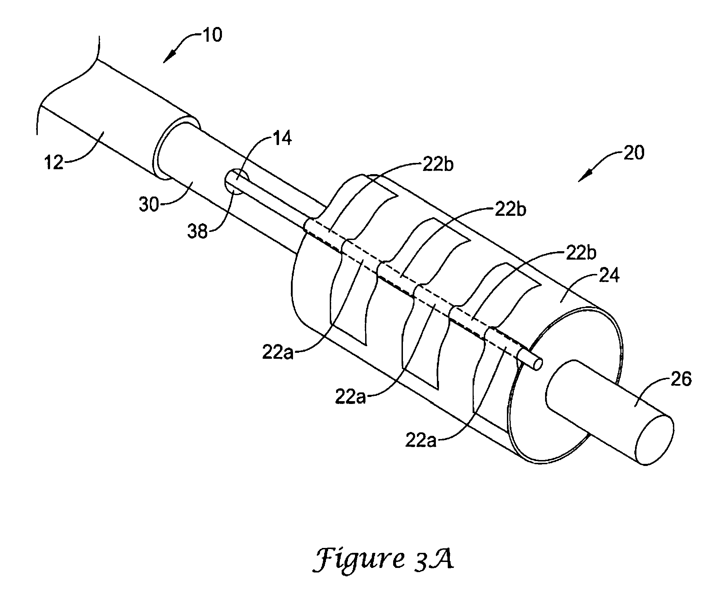

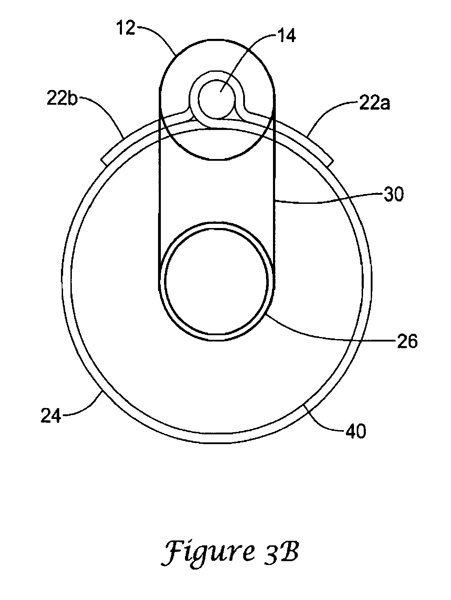

Figure 3 is illustrative of one of several ways in which the actuation element 14 may engage a containment element 24 when the actuation element is in a first position. Offset flaps 22A and 22B along the edge of containment element 24 may be folded back to create interdigitated loops through which actuation element 14 may pass.

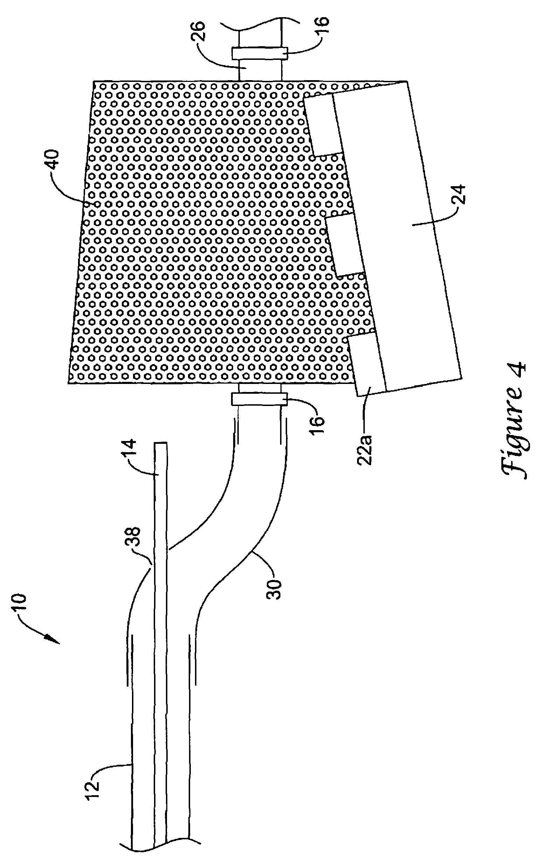

In Figure 4, the filter 40 has been deployed from containment element 24 by withdrawing the actuation element 14 from a first position to a second position, thereby disengaging the actuation element 14 from the loops formed by flaps 22. Once the containment element 24 has been released along the join line between its halves, the filter is free to expand as shown. In some embodiments, the filter 40 may include struts (not shown) which are outwardly biased to urge the filter to expand once released from the containment element 24.

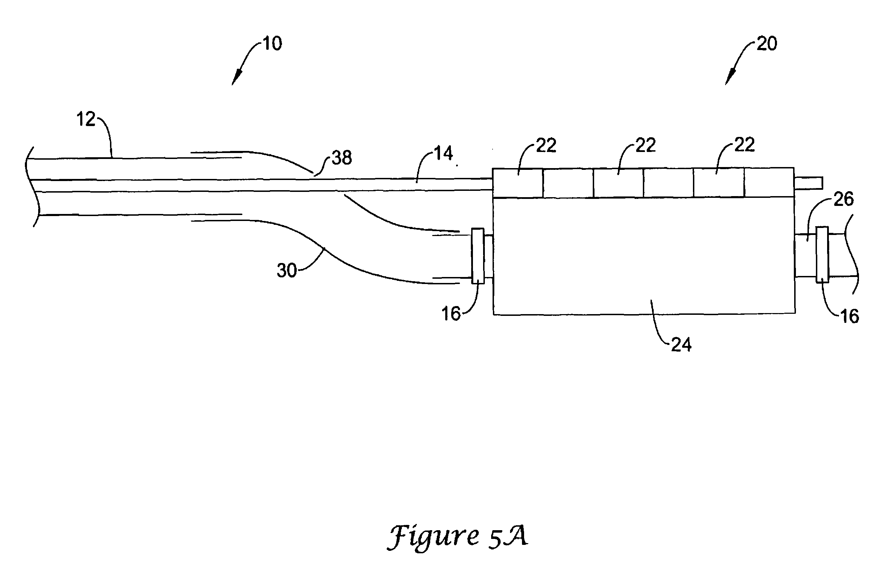

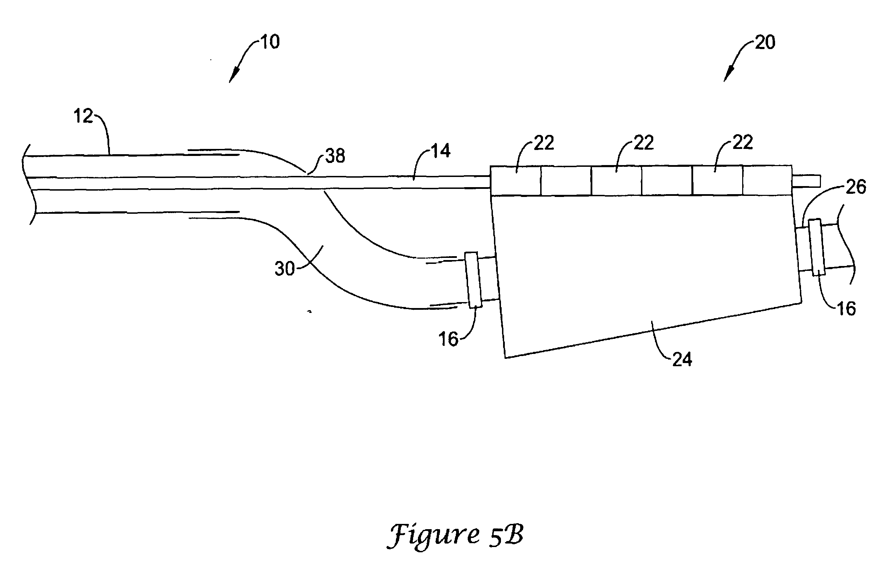

The embodiments of Figures 5A and 5B are similar to those of Figures 2A and 2B except that the offset coupling region 30 and the support segment 26 have been formed from the distal portion of guide member 12 rather than comprising separate items and the support segment 26 is located adjacent to the medical device and optionally in contact with the containment element 24.

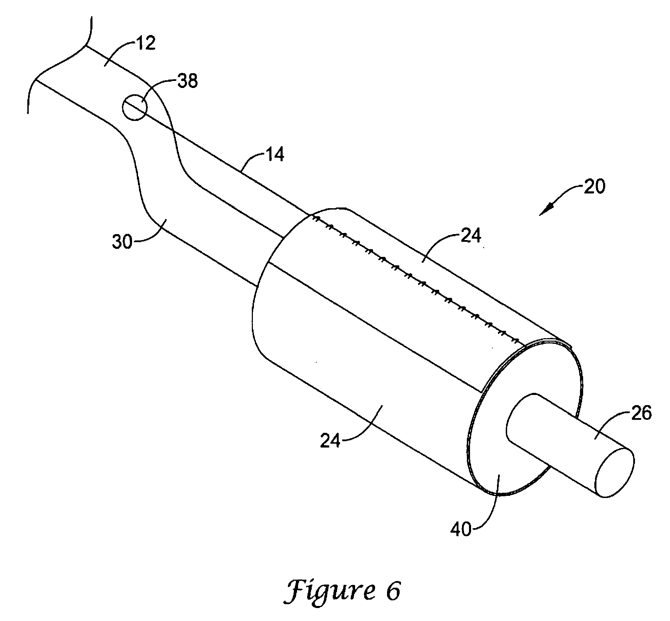

Figure 6 is yet another embodiment in which the offset coupling region 30 and the support segment 26 have been formed from the distal portion of guide member 12 rather than comprising separate items. In addition, the engagement between the actuation element 14 and the containment element 24 resembles a sewn seam in which the actuation element alternately enters and exits apertures formed along the folded edges of the containment element 24 thus bridging the gap and completing the containment while the actuation element is in a first position.

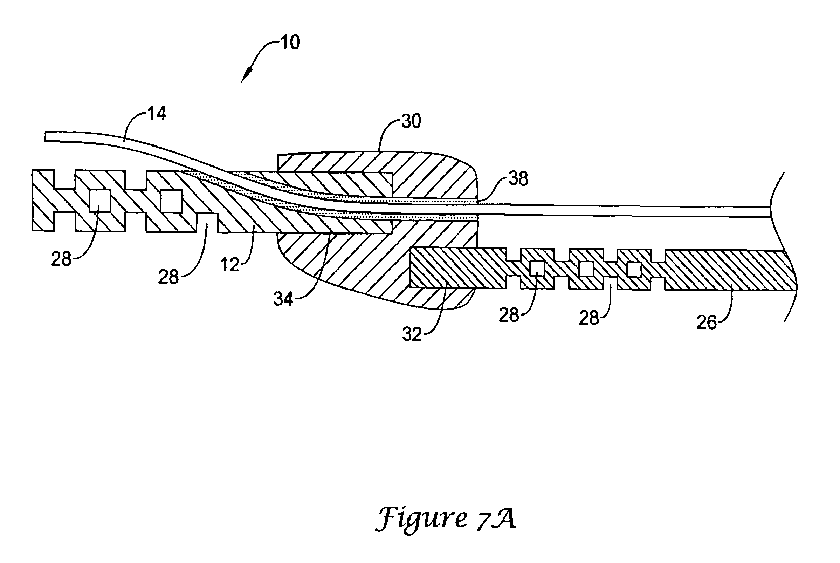

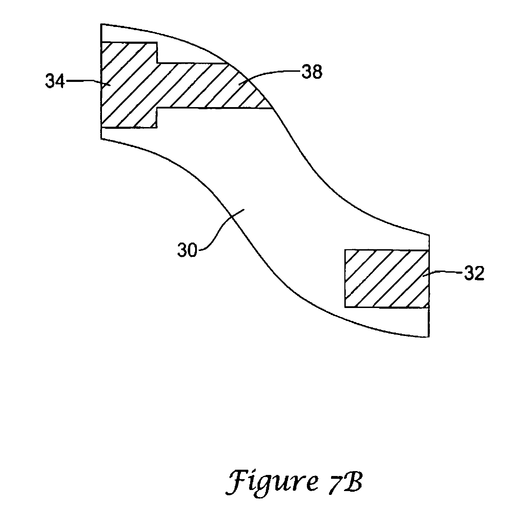

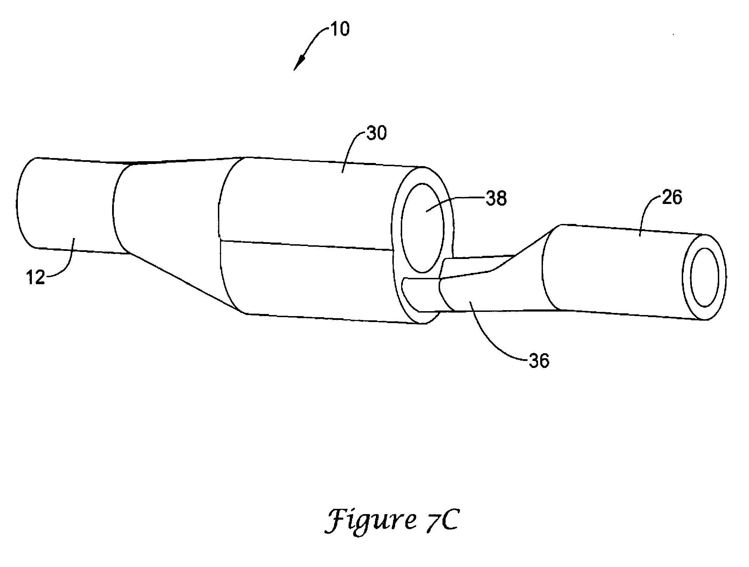

Figures 7A - 7C illustrate various additional configurations which may serve as the offset coupling region 30. In addition, Figure 7A illustrates an embodiment in which both the guide member 12 and the support segment 26 have been partially relieved near the offset coupling region 30 to impart additional flexibility to that region thereby providing a smoother transition as the medical deployment system is maneuvered toward the deployment site. The partial relief may be provided by a series of holes, slots, or grooves 28. The embodiment of Figure 7A includes a molded, cast, or machined offset coupling region 30 which may be formed from metal, plastic, or a composite material such as a filled polymer. As illustrated, the offset coupling region 30 has separate lumens 32, 34 to accept the support segment 26 and guide member 12 respectively. In addition, lumen aperture 38 allows free passage of actuation element 14 which is shown as entering a solid guide member 12 just proximal of the offset coupling region 30. The offset coupling region 30 configuration of Figure 7B also may be molded, cast, or machined from metal, plastic, or a composite material such as a filled polymer. It includes separate lumens 32,34 to accept the support segment 26 and guide member 12 respectively, as well as a lumen aperture 38 to allow free passage of actuation element 14. Figure 7C illustrates an embodiment in which the proximal end 36 of a tubular support segment 26 has been crimped prior to insertion into the offset coupling region 30. This embodiment may allow the actuation element 14 to exit the aperture 38 along a substantially linear axial extension of a guide member lumen while remaining relatively near and parallel to the support segment 26. Such a configuration may be desirable, for example, if the containment element 24 is attached to the support segment and the engagement between the actuation element 14 and the containment element 24 is adjacent to the support element. Other arrangements and configurations of the offset coupling region, the support segment, the containment element, the actuation element and the medical device will be apparent to those skilled in the art.

In some embodiments, the support segment is generally parallel to and offset from the guide member while in other embodiments, the support segment is angled somewhat toward or away from a line corresponding to the extended guide member. In other embodiments, the majority of the support segment is located distal of the distal end of the guide member. In some embodiments, the offset coupling region connects the guide member to the support segment without intervening elements such as sleeves which accommodate one or both of the distal end of guide member and the proximal end of the offset coupling region and the distal end of the offset coupling region and the distal end of the support segment. It will be appreciated that the coupling of the offset coupling region to the guide member and the support segment respectively may be accomplished in several equivalent ways and that some of the coupling patterns may depart from the generally collinear transitions illustrated herein for convenience. In some embodiments, the offset coupling region comprises a separate coupling element, typically formed from metal, polymer, or reinforced polymer, while in other embodiments, it may be formed, at least in part, from one or both of the guide member and the support segment.

In those embodiments in which a separate coupling element or region is employed, the offset coupling element often comprises a first lumen adapted to receive the guide member and a second lumen adapted to receive the support segment. In addition to the sequential arrangements shown in the various figures, it will be appreciated that the elements may be joined to each other in alternate configurations which also allow the actuation element to pass generally directly from the guide member to engage with the containment element along a line generally extending coaxially from the guide member. The offset coupling region may be disposed near the distal end of the guide member such that the actuation element exits the guide member directly without the need for a separate aperture. In some embodiments, the offset coupling region between the guide member and the support segment may be adapted for rotation about an axis generally coincident with the actuation member and passing through the distal end of the elongated guide member. The guide member, offset coupling region, and support segment, along with any intervening elements, may be joined by adhesives, soldering, welding, crimping, and the like without departing from the spirit of the invention. One or both of the guide member and the support segment may have a reduced cross-sectional area where it is received by the offset coupling element. In some embodiments, the offset coupling region may comprise one or more tapered segments.

In some embodiments, the offset coupling region includes an aperture formed in the offset coupling region and generally aligned with an axial extension of a lumen associated with the guide member such that the actuation element passes through the lumen and the aperture in a straight line. Having passed through the lumen and the aperture, the actuation element generally continues along that same straight line to the point or points of engagement with the containment element when the actuation element is in the first position. In some embodiments, the offset coupling region comprises a double-curved portion of the guide member and the support segment comprises a further distal portion of the guide member. In yet other embodiments, the medical device is longitudinally constrained with regard to motion along the support section, for example, by proximal and/or distal stops. The offset between the guide member and the support segment may serve as a proximal stop to limit translation of the medical device in the proximal direction.

In some embodiments, the actuation element exits the guide member at the proximal end of the guide member. In other embodiments, the actuation element is disposed at least partially within a lumen of the guide member and extends distally from the guide member in the offset coupling region. In yet other embodiments, actuation element enters a lumen of the guide member near the distal end of the guide member in the manner of a single operator exchange catheter and extends distally from the guide member in the offset coupling region.

In some embodiments, the containment element at least partially envelopes at least one of the medical device and the support segment when the actuation element is in the first position. In one configuration, the containment element has one or more apertures formed therein and the actuation element engages one or more apertures in the containment element in the first position and disengages from at least a majority of the one or more apertures in the containment element in the second position. In addition to simple containment, the containment element may provide a constraining force to the medical device when the actuation element is in the first position. When the actuation element is moved from the first position to the second position, the containment and/or the constraining force are removed or otherwise inactivated. It will be appreciated that the transition from the first position of the actuation element to the second position of the actuation element may occur with either distal or proximal motion of the actuation element depending upon the configuration of the points of engagement between the actuation element and the containment element. In other embodiments, the transition from the first position of the actuation element to the second position of the actuation element may occur with rotation of the actuation element or with some combination of translation and rotation.

In some embodiments, it may be desirable to relieve the guide member and/or the support segment by adding holes, slots, or partial or complete grooves to provide increased flexibility, especially in the vicinity of the offset coupling region. Such relief is believed useful for maintaining a smooth transitional curve in the region as the guide member and the support segment are guided to the site at which the medical device is to be deployed. The holes, slots, or grooves may be uniformly spaced or may be nonuniformly spaced to achieve the desired flexibility profile along the delivery system. One of skill in the art will appreciate that either the guide member or the support segment may be solid or hollow along it respective entire length and that portions of one or both may be solid or hollow in alternate embodiments. For example, in some embodiments, the guide member comprises a lumen adjacent to the proximal end of the offset coupling region and a portion of the actuation member resides within the lumen. The actuation member may exit the guide member proximal the offset coupling region.

Various modifications and alterations of this invention will become apparent to those skilled in the art without departing from the scope of the appended claims , and it should be understood that this invention is not to be unduly limited to the illustrative embodiments set forth hereinabove.

## Claims

1. A medical device deployment system comprising:
an elongated guide member (12) having a proximal end, a distal end, and a lumen at least partially therethrough;
a support segment (26) attached near the distal end of the elongated guide member (12),
a medical device disposed adjacent to the support segment (26);
a containment element (24) disposed about at least a portion of the medical device;
an actuation element (14) having a proximal end and a distal end and a first position and a second position, wherein the distal end of the actuation element (14) engages at least a portion of the containment element (24) in the first position and is at least partially disengaged from the containment element (24) in the second position; and
**characterised in that** there is an offset coupling region (30) between the guide member (12) and the support segment (26), wherein the actuation element (14) extends beyond the distal end of the guide member (12) and generally coaxial with it to engage the containment element (24) at one or more points in the first position.

2. The medical device deployment system of claim 1, wherein the support segment is generally parallel to and offset from the guide member.

3. The medical device deployment system of claim 1, wherein the majority of the support segment is located distal of the distal end of the guide member.

4. The medical device deployment system of claim 1, wherein the offset coupling region connects the guide member to the support segment.

5. The medical device deployment system of claim 1, wherein the offset coupling region includes an aperture formed therein generally aligned with an axial extension of a lumen associated with the guide member,
further wherein the actuation element passes through the lumen and the aperture.

6. The medical device deployment system of claim 5, wherein the engagement between the actuation element in the first position and the containment element occurs generally along an axial extension of the lumen associated with the guide member.

7. The medical device deployment system of claim 1, wherein the offset coupling region comprises a separate coupling element.

8. The medical device deployment system of claim 7, wherein the separate coupling element is formed from at least one of metal, polymer, or reinforced polymer.

9. The medical device deployment system of claim 7, wherein the offset coupling element comprises a first lumen adapted to receive the guide member and a second lumen adapted to receive the support segment.

10. The medical device deployment system of claim 9, wherein one or both of the guide member and the support segment has a reduced cross-sectional area where it is received by the offset coupling element.

11. The medical device deployment system of claim 1, wherein the offset coupling region comprises a double-curved portion of the guide member and the support segment comprises a further distal portion of the guide member.

12. The medical device deployment system of claim 11, wherein support segment is generally parallel to the guide member.

13. The medical device deployment system of claim 11, wherein the offset coupling region includes an aperture formed in the offset coupling region and generally aligned with an extended axis of a lumen associated with the guide member proximal of the offset coupling region,
further wherein the actuation element passes through the lumen and the aperture formed in the offset coupling region.

14. The medical device deployment system of claim 1, wherein the medical device is longitudinally constrained with regard to motion along the support section.

15. A medical device deployment system of claim 1, wherein the actuation element is disposed at least partially within a lumen of the guide member and extends distally from the guide member in the offset coupling region.

## Patentansprüche

1. Einsatzsystem für eine medizinische Vorrichtung mit:
einem länglichen Führungselement (12) mit einem proximalen Ende, einem distalen Ende und einem sich mindestens teilweise dort hindurch erstreckenden Lumen;
einem Haltesegment (26), das nahe des distalen Endes des länglichen Führungselements (12) befestigt ist;
einer medizinischen Vorrichtung, die benachbart zum Haltesegment (26) angeordnet ist;
einem Zurückhalteelement (24), das über mindestens einen Abschnitt der medizinischen Vorrichtung angeordnet ist;
einem Betätigungselement (14) mit einem proximalen Ende und einem distalen Ende und einer ersten Position und einer zweiten Position, wobei das distale Ende des Betätigungselements (14) in der ersten Position mit mindestens einem Abschnitt des Zurückhalteelements (24) in Eingriff steht und in der zweiten Position mindestens teilweise vom Zurückhalteelement (24) gelöst ist; und
**dadurch gekennzeichnet, dass** es
einen Versatzkupplungsbereich (30) zwischen dem Führungelement (12) und dem Haltesegment (26) gibt, wobei sich das Betätigungselement (14) über das distale Ende des Führungselements (12) hinaus und im Wesentlichen koaxial damit erstreckt, um in der ersten Position mit dem Zurückhalteelement (24) an einem oder mehreren Punkten in Eingriff zu treten.

2. Einsatzsystem für eine medizinische Vorrichtung nach Anspruch 1, wobei das Haltesegment im Wesentlichen parallel und versetzt zum Führungselement ist.

3. Einsatzsystem für eine medizinische Vorrichtung nach Anspruch 1, wobei der größere Teil des Haltesegments distal vom distalen Ende des Führungselements angeordnet ist.

4. Einsatzsystem für eine medizinische Vorrichtung nach Anspruch 1, wobei der Versatzkupplungsbereich das Führungselement mit dem Haltesegment verbindet.

5. Einsatzsystem für eine medizinische Vorrichtung nach Anspruch 1, wobei der Versatzkupplungsbereich eine darin ausgebildete Öffnung aufweist, die im Wesentlichen mit einer axialen Ausdehnung eines Lumens ausgerichtet ist, das mit dem Führungselement in Zusammenhang steht, wobei das Betätigungselement ferner durch das Lumen und die Öffnung geht.

6. Einsatzsystem für eine medizinische Vorrichtung nach Anspruch 5, wobei in der ersten Position der Eingriff zwischen dem Betätigungselement und dem Zurückhalteelement im Wesentlichen längs einer axialen Ausdehnung des mit dem Führungselement in Zusammenhang stehenden Lumens stattfindet.

7. Einsatzsystem für eine medizinische Vorrichtung nach Anspruch 1, wobei der Versatzkupplungsbereich ein getrenntes Kupplungselement aufweist.

8. Einsatzsystem für eine medizinische Vorrichtung nach Anspruch 7, wobei das getrennte Kupplungselement aus Metall und/oder einem Polymer und/oder einem verstärkten Polymer ausgebildet ist.

9. Einsatzsystem für eine medizinische Vorrichtung nach Anspruch 7, wobei das Versatzkupplungselement ein erstes Lumen, das eingerichtet ist, das Führungselement aufzunehmen, und ein zweites Lumen aufweist, das eingerichtet ist, das Haltesegment aufzunehmen.

10. Einsatzsystem für eine medizinische Vorrichtung nach Anspruch 9, wobei das Führungselement und/oder das Haltesegment einen Bereich mit reduziertem Querschnitt aufweist, wo es durch das Versatzkupplungselement aufgenommen wird.

11. Einsatzsystem für eine medizinische Vorrichtung nach Anspruch 1, wobei der Versatzkupplungsbereich einen doppelt gekrümmten Abschnitt des Führungselements aufweist und das Haltesegment einen weiteren distalen Abschnitt des Führungselements aufweist.

12. Einsatzsystem für eine medizinische Vorrichtung nach Anspruch 11, wobei Haltesegment im Wesentlichen parallel zum Führungselement ist.

13. Einsatzsystem für eine medizinische Vorrichtung nach Anspruch 11, wobei der Versatzkupplungsbereich eine Öffnung aufweist, die im Versatzkupplungsbereich ausgebildet ist und im Wesentlichen mit einer verlängerten Achse eines Lumens ausgerichtet ist, das proximal vom Versatzkupplungsbereich mit dem Führungselement in Zusammenhang steht,
wobei das Betätigungselement ferner durch das Lumen und die im Versatzkupplungsbereich ausgebildete Öffnung geht.

14. Einsatzsystem für eine medizinische Vorrichtung nach Anspruch 1, wobei die medizinische Vorrichtung hinsichtlich einer Bewegung längs des Halteabschnitts longitudinal eingeschränkt ist.

15. Einsatzsystem für eine medizinische Vorrichtung nach Anspruch 1, wobei das Betätigungselement mindestens teilweise in einem Lumen des Führungselements angeordnet ist und sich distal vom Führungselement in dem Versatzkupplungsbereich erstreckt.

## Revendications

1. Système de mise en place d'un dispositif médical, comprenant :
un élément de guidage oblong (12) avec une extrémité proximale, une extrémité distale, et au moins partiellement traversé par une lumière ;
un segment de support (26) fixé à proximité de l'extrémité distale de l'élément de guidage oblong (12) ;
un dispositif médical adjacent au segment de support (26) ;
un élément d'enceinte (24) disposé autour d'au moins une partie du dispositif médical ;
un élément actionneur (14) présentant une extrémité proximale et une extrémité distale et une première position et une deuxième position, l'extrémité distale dudit élément actionneur (14) étant engagée dans au moins une partie de l'élément d'enceinte (24) dans la première position et étant au moins partiellement désengagée de l'élément d'enceinte (24) dans la deuxième position ;
**caractérisé**
**en ce qu'**est présentée une zone de raccord en décalage (30) entre l'élément de guidage et le segment de support (26), l'élément actionneur (14) s'étendant au-delà de l'extrémité distale de l'élément de guidage (12) et sensiblement coaxialement à celui-ci pour s'engager dans l'élément d'enceinte (24) sur un ou plusieurs points dans la première position.

2. Système de mise en place d'un dispositif médical selon la revendication 1, où le segment de support est sensiblement parallèle à l'élément de guidage et décalé de celui-ci.

3. Système de mise en place d'un dispositif médical selon la revendication 1, où la plus grande partie du segment de support est distale par rapport à l'extrémité distale de l'élément de guidage.

4. Système de mise en place d'un dispositif médical selon la revendication 1, où la zone de raccord en décalage raccorde l'élément de guidage au segment de support.

5. Système de mise en place d'un dispositif médical selon la revendication 1, où la zone de raccord en décalage comporte une ouverture formée à l'intérieur de celle-ci, sensiblement alignée avec une extension axiale d'une lumière associée à l'élément de guidage, et où l'élément actionneur traverse en outre la lumière et l'ouverture.

6. Système de mise en place d'un dispositif médical selon la revendication 5, où l'engagement de l'élément actionneur dans l'élément d'enceinte dans la première position est sensiblement réalisé le long d'une extension axiale de la lumière associée à l'élément de guidage.

7. Système de mise en place d'un dispositif médical selon la revendication 1, où la zone de raccord en décalage comporte un élément de raccord séparé.

8. Système de mise en place d'un dispositif médical selon la revendication 7, où l'élément de raccord séparé est constitué d'au moins un matériau sélectionné parmi un métal, un polymère, ou un polymère renforcé.

9. Système de mise en place d'un dispositif médical selon la revendication 7, où l'élément de raccord en décalage comporte une première lumière prévue pour recevoir l'élément de guidage et une deuxième lumière prévue pour recevoir le segment de support.

10. Système de mise en place d'un dispositif médical selon la revendication 9, où soit l'élément de guidage, soit le segment de support, soit les deux, ont une surface de section transversale réduite à la réception par l'élément de raccord en décalage.

11. Système de mise en place d'un dispositif médical selon la revendication 1, où la zone de raccord en décalage comporte une partie de l'élément de guidage doublement incurvée, et où le segment de support comporte une autre partie distale de l'élément de guidage.

12. Système de mise en place d'un dispositif médical selon la revendication 11, où le segment de support est sensiblement parallèle à l'élément de guidage.

13. Système de mise en place d'un dispositif médical selon la revendication 11, où la zone de raccord en décalage comporte une ouverture formée dans la zone de raccord en décalage et sensiblement alignée avec un axe étendu d'une lumière associée à l'élément de guidage proximal de la zone de raccord en décalage,
et où l'élément actionneur traverse en outre la lumière et l'ouverture formée dans la zone de raccord en décalage.

14. Système de mise en place d'un dispositif médical selon la revendication 1, où le dispositif médical est retenu longitudinalement pour empêcher un déplacement le long de la section de support.

15. Système de mise en place d'un dispositif médical selon la revendication 1, où l'élément actionneur est disposé au moins partiellement dans une lumière de l'élément de guidage, avec une extension distale de l'élément de guidage vers la zone de raccord en décalage.
